## Europäisches Patentamt
## European Patent Office
## Office européen des brevets

(11) Veröffentlichungsnummer: **0 084 151**
**A1**

(12) # EUROPÄISCHE PATENTANMELDUNG

(21) Anmeldenummer: 82111823.9

(22) Anmeldetag: 20.12.82

(51) Int. Cl.³: **C 07 C 11/02**
**C 07 C 1/04**

(30) Priorität: 19.01.82 DE 3201457

(43) Veröffentlichungstag der Anmeldung:
27.07.83 Patentblatt 83/30

(84) Benannte Vertragsstaaten:
BE DE FR GB IT NL

(71) Anmelder: BASF Aktiengesellschaft
Carl-Bosch-Strasse 38
D-6700 Ludwigshafen(DE)

(72) Erfinder: Reich, Hans Heiner, Dr.
Kranichstrasse 11
D-6700 Ludwigshafen(DE)

(72) Erfinder: Bittler, Knut, Dr.
Kardinal-Wendel-Strasse 54
D-6720 Speyer(DE)

(72) Erfinder: Ostertag, Werner, Dr.
Oberer-Bergel-Weg 2
D-6718 Gruenstadt(DE)

(72) Erfinder: Ertl, Gert, Dr.
Malvenweg 2
D-6800 Mannheim(DE)

(54) Verfahren zur Herstellung von Olefinen.

(57) Die vorliegende Erfindung betrifft ein Verfahren zur Herstellung von Olefinen durch thermische Spaltung von Kohlenwasserstoffen in Gegenwart von Wasserdampf in einem indirekt beheizten Röhrenspaltofen, bei dem man für die thermische Spaltung Kohlenwasserstoffe einsetzt, die durch Umsetzung von Kohlenoxid und Wasserstoff an Rutheniumträgerkatalysatoren hersgestellt worden sind.

EP 0 084 151 A1

BASF Aktiengesellschaft                    O.Z. 0050/35691

## Verfahren zur Herstellung von Olefinen

Die vorliegende Erfindung betrifft ein Verfahren zur Herstellung von Olefinen, insbesondere Ethylen, bei dem zunächst Kohlenoxid und Wasserstoff an Rutheniumkatalysatoren zu Kohlenwasserstoffen umgesetzt werden und anschließend die erhaltenen Kohlenwasserstoffe in Gegenwart von Wasserdampf thermisch gespalten werden.

Es ist bekannt, Olefine wie Ethylen und Propylen durch thermische Spaltung von Erdölfraktionen wie Benzinfraktionen (Naphtha), Kerosin oder Gasöl in Gegenwart von Wasserdampf im Röhrenspaltofen (Röhrenspaltung) herzustellen (vgl. Ullmanns Encyklopädie der technischen Chemie, Dritte Auflage, Ergänzungsband, 1970, Seiten 44 bis 50). Da jedoch davon auszugehen ist, daß in der Zukunft mit einer Stagnation des Aufkommens oder sogar mit einer Verknappung von Erdöl und somit der für die Herstellung von Olefinen durch Röhrenspaltung als Einsatzstoffe verwendeten Erdölfraktionen zu rechnen ist, besteht Bedarf nach Verfahren zur Olefinherstellung, bei denen anstelle von Erdölfraktionen Kohlenwasserstoffeinsatzgemische verwendet werden, die auf der Basis von Kohle als fossiler Kohlenstoffträger erzeugt werden können.

Es ist bereits bekannt, daß man ausgehend von Kohle Kohlenwasserstoffgemische herstellen kann, indem man zunächst aus Kohle ein Kohlenoxid/Wasserstoffgemisch, das sogenannte Synthesegas, herstellt und anschließend das erhaltene Synthesegas an heterogenen Katalysatoren, die vorwiegend aus Metallen der 8. Gruppe des Periodensystems bestehen, umsetzt, wobei man zu Gemischen aus gesättigten und ungesättigten Kohlenwasserstoffen sowie sauerstoffhaltigen Kohlenwasserstoffverbindungen gelangen kann.

Ste/P

Dieses auch als Fischer-Tropsch-Synthese bezeichnete Verfahren ist jedoch wegen seiner mangelnden Selektivität und wegen seines relativ niedrigen thermischen Wirkungsgrades nicht zufriedenstellend für die Herstellung von Kohlenwasserstoffen, die als Einsatzstoffe für die Olefinherstellung durch Röhrenspaltung Verwendung finden sollen. Diese Einsatzstoffe sollten nämlich, um eine möglichst große Olefinausbeute zu liefern, einen hohen Anteil an geradkettigten Kohlenwasserstoffen aufweisen. Außerdem sollte das Molekulargewicht der für die Röhrenspaltung als Einsatzstoffe verwendeten Kohlenwasserstoffe eine obere Grenze von ca. 700 bis 1000 nicht überschreiten, um eine leichte Dosierbarkeit in einer technischen Anlage zu gewährleisten. Gleichzeitig sollte bei der Herstellung dieser Kohlenwasserstoffe die Bildung sauerstoffhaltiger Verbindungen möglichst vermieden werden, da die bei der Fischer-Tropsch-Synthese gewöhnlich beobachtete Kuppelproduktion derartiger organischer Verbindungen wie Alkohole, Aldehyde, Ketone, Ester, Säuren entweder eine komplizierte Aufarbeitung oder aber Umweltprobleme, z.B. eine aufwendige Abwasseraufbereitung, mit sich bringt. Eine weitere wichtige Anforderung bei der Herstellung der Kohlenwasserstoffe durch die katalytische Umsetzung von Kohlenoxid und Wasserstoff bestand darin, daß die Bildung von den unerwünschten Nebenprodukten Methan und Kohlendioxid möglichst unterdrückt wird.

Es wurde nun ein vorteilhaftes Verfahren gefunden zur Herstellung von Olefinen durch thermische Spaltung von Kohlenwasserstoffen in Gegenwart von Wasserdampf in einem indirekt beheizten Röhrenspaltofen, welches dadurch gekennzeichnet ist, daß man für die thermische Spaltung Kohlenwasserstoffe einsetzt, die durch Umsetzung von Kohlenoxid und Wasserstoff an Rutheniumträgerkatalysatoren hergestellt worden sind.

Bei dem erfindungsgemäßen Verfahren können durch die Umsetzung von Kohlenoxid und Wasserstoff Kohlenwasserstoffe erhalten werden, die zu mehr als 70 % aus geradkettigen Kohlenwasserstoffen bestehen und gleichzeitig genügend niedrige Molekulargewichte aufweisen, um eine leichte Dosierbarkeit bei ihrer Weiterverwendung als Einsatzstoff für die thermische Spaltung zu Olefinen zu ermöglichen. Im Vergleich zur Herstellung von Olefinen durch thermische Spaltung von Naphtha können nach dem erfindungsgemäßen Verfahren um bis zu 40 % höhere Ethylenausbeuten erzielt werden.

Ein weiterer Vorteil des erfindungsgemäßen Verfahrens besteht darin, daß unerwünschte Nebenprodukte wie $CO_2$, sauerstoffhaltige Verbindungen und Methan bei der katalytischen Umsetzung von Kohlendioxid und Wasserstoff an Rutheniumträgerkatalysatoren nur in sehr geringen Mengen gebildet werden. Dies war überraschend, da aus F. Bellstedt, Dissertation Universität Karlsruhe, 1971 und M.A. Vannice, Catalytic Review, Band 14 (2), Seite 172, Figur 9, bekannt ist, daß metallisches Ruthenium als Katalysator bei der Umsetzung von Kohlenoxid und Wasserstoff in sehr starkem Maße zur Methanbildung führt - so wurde bei 140°C eine Selektivität bezüglich der Methanbildung von bis zu 24 % gefunden - und daß metallisches Ruthenium unter den Metallen der 8. Nebengruppe des Periodensystems der Elemente der aktivste Methanisierungskatalysator ist.

Darüber hinaus wurde bisher (vgl. z.B. H. Pichler, Advances in Catalysis, Band 4, 1952, Seite 291) feinverteiltes metallisches Ruthenium in trägerfreier Form als der optimale Rutheniumkatalysator angesehen, der weder durch Promotoren noch durch Träger in seiner Wirksamkeit verbessert werden kann. Es war daher überraschend, daß bei der erfindungsgemäßen Verwendung der Rutheniumträgerkatalysatoren für die Umsetzung von Kohlenoxid und Wasserstoff die Methanbildung sehr stark reduziert wird und daß Rutheniumträgerkatalysatoren gleichzeitig eine hohe Aktivität in Bezug auf die Bildung von geradkettigen höheren Kohlenwasserstoffen aufweisen.

Für die erfindungsgemäße Umsetzung von Kohlenoxid und Wasserstoff zu Kohlenwasserstoffen werden Rutheniumträgerkatalysatoren verwendet. Als Trägermaterial kommen z.B. Kieselgel, Titandioxid, Thoriumdioxid, Aluminiumoxid, natürliche Silikate, Aktivkohle oder Zeolithe oder deren Mischungen in Betracht. Vorzugsweise wird Kieselgel als Trägermaterial verwendet. Im allgemeinen weisen die Rutheniumträgerkatalysatoren Rutheniumgehalte von 0,01 bis 40 Gew.-%, vorzugsweise 0,1 bis 20 Gew.-%, insbesondere 1 bis 5 Gew.-%, bezogen auf den Trägerkatalysator auf. Man kann die Rutheniumträgerkatalysatoren ohne weitere Zusätze verwenden. Es kann jedoch vorteilhaft sein, solche Trägerkatalysatoren zu verwenden, die neben Ruthenium als Co-Katalysatoren noch Magnesium, Zink, Mangan, Praseodym, Titan, Molybdän und Wolfram oder deren Mischungen enthalten. Der Gehalt der Trägerkatalysatoren an den Co-Katalysatoren beträgt im allgemeinen 0,01 bis 20 Gew.-%, vorzugsweise 0,1 bis 10 Gew.-%, insbesondere 1 bis 5 Gew.-%, bezogen auf den Trägerkatalysator.

Das Aufbringen der katalytisch aktiven Substanzen auf das Trägermaterial kann beispielsweise durch Tränken, Auffällen, Ionenaustausch oder auch durch Plasmaspritzen erfolgen. Dabei wird Ruthenium zweckmäßig in Form der Rutheniumverbindungen, z.B. als Ruthenium-Komplexe, -Salze und/oder -Oxide aufgetragen, wobei vorteilhaft die Chloride, Bromide Jodide, Nitrate und Carboxylate verwendet werden. Für das Auftragen der Co-Katalysatoren kommen

beispielsweise die Oxide und vorzugsweise die Salze und/oder Komplexe der entsprechenden Metalle in Betracht.

Falls die Aktivität des Rutheniumträgerkatalysators bei der katalytischen Umsetzung von Kohlenoxid und Wasserstoff nachläßt, wird er zweckmäßig durch Regenerieren reaktiviert. Dies kann z.B. dadurch geschehen, daß man den gebrauchten Trägerkatalysator mit einem organischen Lösungsmittel, z.B. Toluol, in Berührung bringt. Eine andere Möglichkeit zur Regenerierung des Trägerkatalysators besteht darin, daß man den gebrauchten Trägerkatalysator bei erhöhten Temperaturen, z.B. bei Temperaturen von 150 bis 400°C, vorzugsweise 180 bis 380°C, solange mit Gemischen von Sauerstoff mit einem inerten Gas, z.B. Stickstoff behandelt, bis im Abgas kein Kohlendioxid mehr nachzuweisen ist. Der Trägerkatalysator wird im allgemeinen vor seiner Verwendung bzw., vor seiner Wiederverwendung nach der Regenerierung mit Wasserstoff einer reduzierenden Behandlung unterworfen.

Für die katalytische Umsetzung von Kohlenoxid und Wasserstoff an den Rutheniumträgerkatalysatoren werden im allgemeinen Temperaturen von 120°C bis 450°C, vorzugsweise 150°C bis 400°C, insbesondere 160°C bis 380°C, sowie Drücke von 10 bis 350 bar, vorzugsweise 50 bis 200 bar, insbesondere 60 bis 150 bar, angewendet.

Für die katalytische Umsetzung werden Kohlenoxid und Wasserstoff im allgemeinen im Molverhältnis 5 : 1 bis 1 : 10, vorzugsweise 2 : 1 bis 5 : 1, insbesondere 1,1 : 1 bis 1 : 2,2, eingesetzt. Mit besonderem Vorteil werden Kohlenoxid/Wasserstoff-Mischungen im Molverhältnis von ca. 1 : 1 und 1 : 2 verwendet.

Die durch die katalytische Umsetzung von Kohlenoxid und Wasserstoff an Rutheniumträgerkatalysatoren hergestellten Kohlenwasserstoffe werden erfindungsgemäß als Einsatzstoffe zur Herstellung von Olefinen, insbesondere Ethylen, durch thermische Spaltung dieser Kohlenwasserstoffe in Gegenwart von Wasserdampf in einem indirekt beheizten Röhrenspaltofen (Steamcracker) verwendet. Im allgemeinen beträgt das Gewichtsverhältnis von Wasserdampf zum eingesetzten Kohlenwasserstoff bei der thermischen Spaltung 0,1 bis 1, vorzugsweise 0,2 bis 0,8, insbesondere 0,3 bis 0,7. Die Verweilszeiten für die Kohlenwasserstoffe in den Spaltrohren des Röhrenspaltofens betragen zweckmäßig 0,05 bis 1 sec., vorzugsweise 0,1 bis 0,5 sec.. Für die bei der thermischen Spaltung der Kohlenwasserstoffe erhaltenen Spaltgase werden im allgemeinen Austrittstemperaturen der Spaltgase aus den Spaltrohren von mehr als 700°C, vorzugsweise von mehr als 750°C, insbesondere von 800 bis 900°C, eingehalten.

Die erhaltenen Spaltgase werden zweckmäßig sofort abgekühlt. Dies kann durch direkte Abkühlung durch Einspritzen von flüssigen Kohlenwasserstoffen oder von Wasser in das heiße Spaltgas erfolgen. Im allgemeinen wird jedoch die Abkühlung der Spaltgase indirekt durchgeführt, indem die Spaltgase durch einen Spaltgaskühler geleitet werden, in welchem die Spaltgase in indirektem Wärmeaustausch mit einem flüssigen Wärmeträger, vorzugsweise Wasser, gekühlt werden.

Die folgenden Beispiele veranschaulichen die Erfindung.

Beispiel 1

240 g wasserfestes Kieselgel (Strangpreßlinge von 4 mm) mit einem Schüttgewicht von 445 g/l und einer Oberfläche

nach Brunnauer, Emmett und Teller (vgl. JACS 60, Seite 309, 1938) von 156 $m^2$/g wurden mit einer Lösung von 91,2 g Magnesiumchloridhydrat in 280 ml vollentsalztem Wasser versetzt. Man ließ das Magnesiumsalz innerhalb von 30 min. bei 70°C und 20 Torr auf das Kieselgelträgermaterial aufziehen und trocknete anschließend 2 Stunden bei 80°C und danach 3 Stunden bei 160°C unter Atmosphärendruck. Schließlich wurde 130 min. bei 860°C getempert und dann abgekühlt. Das erhaltene Material wurde mit einer Lösung von 36 g $RuCl_3$ in 240 ml vollentsalztem Wasser getränkt, und das feuchte Material wurde bei 70°C und 20 Torr entwässert. Nach einer jeweils 2,5-stündigen Trocknung bei 80°C und 160°C wurde der erhaltene Katalysator in einem Rohrreaktor bei 200°C bei Atmosphärendruck mit 90 1 Wasserstoff pro Stunde reduziert. Der erhaltene Trägerkatalysator hatte einen Rutheniumgehalt von 4,8 Gew.-% und einen Magnesiumgehalt von 4,5 Gew.-%.

80 ml dieses Trägerkatalysators wurden in einen Autoklaven mit innerem Gasumlauf (System Berty) eingefüllt, und bei 260°C und 100 bar wurde kontinuierlich Synthesegas zugeführt, dessen $CO/H_2$-Molverhältnis 1 : 2 betrug. 300 Nl/h Abgas wurden aus dem Autoklaven abgezogen, und 20,02 g pro Stunde an kondensierbaren Kohlenwasserstoffen dem Autoklaven entnommen. Das Autoklavenkreisgas enthielt 1,56 Vol.-% Methan und weniger als 0,1 Vol.-% $CO_2$. Der Anteil an sauerstoffhaltigen Verbindungen in der wäßrigen Phase des Autoklavenaustrags lag unter 2 Gew.-%.

900 g des bei der katalytischen Umsetzung im Autoklaven erhaltenen Kohlenwasserstoffgemischs wurden nach Vermischen mit Wasserdampf (Gewichtsverhältnis von Wasserdampf zu Kohlenwasserstoffgemisch von 0,48 : 1) in einem Laborröhrenspaltofen thermisch gespalten. Die Austrittstemperatur des aus dem Spaltrohr austretenden Spaltgases betrug 813°C. Die Zusammensetzung des aus dem Spaltofen erhaltenen Kohlenwasserstoff-Produktgemisches ist in der Tabelle aufgeführt.

Vergleichsbeispiel

Man verfuhr wie im 3. Absatz von Beispiel 1 beschrieben, wobei jedoch anstelle des durch die katalytische Umsetzung im Autoklaven erhaltenen Kohlenwasserstoffgemischs 900 g Leichtbenzin (Naphtha) (Siedebereich 45 bis 158°C), wie es üblicherweise in großtechnischen Ethylenanlagen eingesetzt wird, verwendet wurden. Die Zusammensetzung des aus dem Spaltofen erhaltenen Kohlenwassetstoff-Produktgemisches ist in der Tabelle aufgeführt. Bei der Spaltung des erfindungsgemäß erhaltenen Kohlenwasserstoffgemischs gemäß Beispiel 1 wurde, wie der Tabelle zu entnehmen ist, gegenüber der Verwendung von Naphtha als Einsatzstoff eine um mehr als 40 % höhere Ethylenausbeute erhalten.

**0084151**

Tabelle

| Komponenten | Beispiel 1 [Gew.-%] | Vergleichs- beispiel [Gew.-%] | Beispiel 2 [Gew.-%] |
|---|---|---|---|
| $CH_4$ | 10,66 | 13,88 | 11,43 |
| $C_2H_6$ | 4,79 | 4,08 | 4,40 |
| $C_2H_4$ | 34,64 | 24,55 | 32,52 |
| $C_2H_2$ | 0,28 | 0,28 | 0,24 |
| $C_3H_8$ | 1,02 | 0,56 | 0,82 |
| $C_3H_6$ | 16,53 | 14,45 | 14,87 |
| $C_3H_4$ | 0,36 | 0,44 | 0,28 |
| $C_4H_{10}$ | 0,20 | 0,88 | 0,20 |
| $C_4H_8$ | 3,52 | 4,91 | 3,67 |
| $1,3-C_4H_6$ | 5,05 | 3,56 | 5,27 |
| $C_5^+$-Kohlenwasser-stoffe, gasförmig | 9,25 | } 31,62 | 11,04 |
| $C_5^+$-Kohlenwasser-stoff, flüssig | 12,58 | | 12,27 |

Beispiel 2

Man verfuhr wie im Beispiel 1 beschrieben, wobei jedoch dem Autoklaven ein Synthesegas zugeführt wurde, dessen $CO/H_2$-Molverhältnis 1 : 1 (statt 1 : 2 gemäß Beispiel 1) betrug. Man erhielt pro Stunde 11,80 g kondensierbare Kohlenwasserstoffe. Das Autoklavenkreisgas war frei von $CO_2$ und Methan. In der wäßrigen Phase des Autoklavenaustrages waren weniger als 2 Gew.-% an organischen Verbindungen gelöst. Die Zusammensetzung des bei der thermischen Spaltung von 900 g des Kohlenwasserstoffgemischs erhaltenen Kohlenwasserstoff-Produktgemischs ist in der Tabelle aufgeführt.

Patentansprüche

1. Verfahren zur Herstellung von Olefinen durch thermische Spaltung von Kohlenwasserstoffen in Gegenwart von Wasserdampf in einem indirekt beheizten Röhrenspaltofen, dadurch gekennzeichnet, daß man für die thermische Spaltung Kohlenwasserstoffe einsetzt, die durch Umsetzung von Kohlenoxid und Wasserstoff an Rutheniumträgerkatalysatoren hergestellt worden sind.

2. Verfahren nach Anspruch 1, dadurch gekennzeichnet, daß die Rutheniumträgerkatalysatoren Rutheniumgehalte von 0,01 bis 40 Gew.-% aufweisen.

3. Verfahren nach Anspruch 1 und 2, dadurch gekennzeichnet, daß die Trägerkatalysatoren neben Ruthenium als Co-Katalysatoren noch Magnesium, Zink, Mangan, Praseodym, Titan, Molybdän und/oder Wolfram enthalten.

4. Verfahren nach Anspruch 1 bis 3, dadurch gekennzeichnet, daß als Trägermaterialien Kieselgel, Aluminiumoxid, natürliche Silikate und/oder Zeolithe verwendet werden.

5. Verfahren nach Anspruch 1 bis 4, dadurch gekennzeichnet, daß Kohlenoxid und Wasserstoff im Molverhältnis 5 : 1 bis 1 : 10 umgesetzt werden.

6. Verfahren nach Anspruch 1 bis 5, dadurch gekennzeichnet, daß die katalytische Umsetzung von Kohlenoxid und Wasserstoff bei Temperaturen von 120 bis 450°C und Drücken von 10 bis 350 bar durchgeführt wird.

Europäisches
Patentamt

**EUROPÄISCHER RECHERCHENBERICHT**

**0084151**

Nummer der Anmeldung

EP 82 11 1823

## EINSCHLÄGIGE DOKUMENTE

| Kategorie | Kennzeichnung des Dokuments mit Angabe. soweit erforderlich. der maßgeblichen Teile | Betrifft Anspruch | KLASSIFIKATION DER ANMELDUNG (Int. Cl. ³) |
|---|---|---|---|
| Y | DE-A-2 527 316  (CONTINENTAL OIL CO.) <br> * Ansprüche * <br> --- | 1,5,6 | C 07 C  11/02 <br> C 07 C  1/04 |
| Y | NL-A-7 712 952  (SHELL) <br> * Ansprüche * <br> --- | 1,5,6 | |
| Y | DE-A-2 518 097  (MOBIL OIL CORP.) <br> * Seite 5, Zeilen 38-43 * <br> --- | 1,2,4-6 | |
| Y | GB-A-2 024 246  (JOHNSON, MATTHEY & CO.) <br> * Ansprüche; Beispiele * <br> --- | 2,4-6 | |
| Y | DE-A-1 643 672  (B.A.S.F.) <br> * Ansprüche * <br><br> ----- | 1 | **RECHERCHIERTE SACHGEBIETE (Int. Cl. ³)** <br><br> C 07 C  11/00 <br> C 07 C  4/00 <br> C 07 C  1/00 <br> C 10 G  9/00 |

Der vorliegende Recherchenbericht wurde für alle Patentansprüche erstellt.

| Recherchenort <br> DEN HAAG | Abschlußdatum der Recherche <br> 24-03-1983 | Prüfer <br> VAN GEYT J.J.A. |
|---|---|---|